(19) Europäisches Patentamt / European Patent Office / Office européen des brevets

(11) **EP 4 563 702 A1**

(12) **EUROPEAN PATENT APPLICATION**
published in accordance with Art. 153(4) EPC

(43) Date of publication:
**04.06.2025 Bulletin 2025/23**

(21) Application number: **23846258.4**

(22) Date of filing: **13.07.2023**

(51) International Patent Classification (IPC):
*C12N 15/57* (2006.01)   *C11D 7/42* (2006.01)
*C12N 1/21* (2006.01)   *C12N 9/50* (2006.01)
*C12N 15/63* (2006.01)   *C12P 21/02* (2006.01)

(52) Cooperative Patent Classification (CPC):
**C11D 3/386; C12N 9/48; C12N 9/50; C12N 15/63;
C12N 15/74; C12P 21/02**

(86) International application number:
**PCT/JP2023/025933**

(87) International publication number:
**WO 2024/024527 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.07.2022   JP 2022118096**

(71) Applicant: **Kao Corporation
Tokyo 103-8210 (JP)**

(72) Inventors:
• **HIOKI, Takahiro
   Wakayama-shi, Wakayama 640-8580 (JP)**
• **TAKAHIRA, Saki
   Wakayama-shi, Wakayama 640-8580 (JP)**
• **KAWAHARA, Akihito
   Wakayama-shi, Wakayama 640-8580 (JP)**

(74) Representative: **Vossius & Partner
Patentanwälte Rechtsanwälte mbB
Siebertstrasse 3
81675 München (DE)**

Remarks:
The complete document including Reference Table(s) and the Sequence Listing(s) can be downloaded from the EPO website

(54) **MUTANT PROTEASE**

(57)   Provided is a mutant protease having improved cleaning performance against protein stains in a high-concentration detergent liquid. A mutant protease consisting of the amino acid sequence of SEQ ID NO: 1 or an amino acid sequence having an identity of at least 90% with SEQ ID NO: 1 and comprising at least one selected from the group consisting of the following amino acid residues (a) to (g): (a) Arg or Lys at a position corresponding to position 191 of SEQ ID NO: 1; (b) Arg or Lys at a position corresponding to position 17 of SEQ ID NO: 1; (c) Ala or Phe at a position corresponding to position 141 of SEQ ID NO: 1; (d) Arg or Lys at a position corresponding to position 243 of SEQ ID NO: 1; (e) Arg or Lys at a position corresponding to position 300 of SEQ ID NO: 1; (f) Arg or Lys at a position corresponding to position 302 of SEQ ID NO: 1; and (g) Arg or Lys at a position corresponding to position 311 of SEQ ID NO: 1.

Figure 1

**Description**

Field of the Invention

The present invention relates to a mutant protease.

Background of the Invention

The purpose of blending a protease in detergents is to decompose protein stains derived from foods, scurf, sweat and the like attached on clothes, eating utensils and the like, thereby improving the removal of the stains. Since the pH of detergent is generally on the alkali side, proteases having an optimum pH on the alkali side have been developed as proteases for detergents. Patent Literature 1 discloses a protease having a molecular weight of about 43 000 and having cleaning properties against composite stains containing protein and lipid. Patent Literatures 2 to 5 disclose mutant proteases obtained by introducing a mutation which makes the protease have improved cleaning performance, stability in liquid detergent, solubility in liquid detergent or stability under acidic conditions.

Patent Literature 1: WO 99/18218
Patent Literature 2: JP-A-2008-212084
Patent Literature 3: JP-A-2010-273672
Patent Literature 4: JP-A-2013-233141
Patent Literature 5: JP-A-2020-145938

Summary of the Invention

The present invention provides a mutant protease consisting of the amino acid sequence of SEQ ID NO: 1 or an amino acid sequence having an identity of at least 90% with SEQ ID NO: 1 and containing at least one selected from the group consisting of the following amino acid residues (a) to (g):

(a) Arg or Lys at a position corresponding to position 191 of SEQ ID NO: 1;
(b) Arg or Lys at a position corresponding to position 17 of SEQ ID NO: 1;
(c) Ala or Phe at a position corresponding to position 141 of SEQ ID NO: 1;
(d) Arg or Lys at a position corresponding to position 243 of SEQ ID NO: 1;
(e) Arg or Lys at a position corresponding to position 300 of SEQ ID NO: 1;
(f) Arg or Lys at a position corresponding to position 302 of SEQ ID NO: 1; and
(g) Arg or Lys at a position corresponding to position 311 of SEQ ID NO: 1.

The present invention also provides a polynucleotide encoding the mutant protease.
The present invention also provides a vector containing the polynucleotide.
The present invention also provides a recombinant *Bacillus* bacterium containing the polynucleotide or the vector.
The present invention also provides a method for producing a mutant protease using the recombinant *Bacillus* bacterium.
The present invention also provides a detergent composition containing the mutant protease.
Further, the present invention provides a method for producing a mutant protease, containing applying at least one selected from the group consisting of the following mutations (A) to (G) to a parent protease consisting of the amino acid sequence of SEQ ID NO: 1 or an amino acid sequence having an identity of at least 90% with SEQ ID NO: 1:

(A) substitution of Arg or Lys for an amino acid residue at position 191 of SEQ ID NO: 1 or a position corresponding thereto;
(B) substitution of Arg or Lys for an amino acid residue at position 17 of SEQ ID NO: 1 or a position corresponding thereto;
(C) substitution of Ala or Phe for an amino acid residue at position 141 of SEQ ID NO: 1 or a position corresponding thereto;
(D) substitution of Arg or Lys for an amino acid residue at position 243 of SEQ ID NO: 1 or a position corresponding thereto;
(E) substitution of Arg or Lys for an amino acid residue at position 300 of SEQ ID NO: 1 or a position corresponding thereto;

(F) substitution of Arg or Lys for an amino acid residue at position 302 of SEQ ID NO: 1 or a position corresponding thereto; and

(G) substitution of Arg or Lys for an amino acid residue at position 311 of SEQ ID NO: 1 or a position corresponding thereto.

The present invention also provides a method for improving the cleaning performance of a protease against protein stains in a high-concentration detergent liquid, containing applying at least one selected from the group consisting of the mutations (A) to (G) to a parent protease consisting of the amino acid sequence of SEQ ID NO: 1 or an amino acid sequence having an identity of at least 90% with SEQ ID NO: 1.

Brief Description of Drawings

Figure 1 illustrates the relative application cleaning power of a mutant protease.
Figure 2 illustrates the improvement of application cleaning power by a mutation at position 191.

Detailed Description of the Invention

As used herein, the term "amino acid residue" means 20 proteinogenic amino acid residues, which are alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysin (Lys or K), methionine (Met or M), phenylalanine (Phe or F), proline (Pro or P), serine (Ser or S), threonine (Thr or T), tryptophane (Trp or W), tyrosine (Tyr or Y) and valine (Val or V).

As used herein, the term "identity of at least 90%" in relation to amino acid sequences or nucleotide sequences refers to an identity of 90% or more, preferably 95% or more, more preferably 96% or more, further more preferably 97% or more, further more preferably 98% or more, and further more preferably 99% or more.

In the present specification, the identity of the nucleotide sequence and the identity of the amino acid sequence are calculated by a Lipman-Pearson method (Science, 1975, 227: 1435-1441). Specifically, the identity is calculated by analysis using the Search homology program of genetic information processing software Genetyx-Win, where the unit size to compare (ktup) is set to 2.

In the present specification, examples of the "amino acid sequence wherein one or several amino acids are deleted, inserted, substituted or added" include amino acid sequences wherein preferably 1 or more and 10 or less, more preferably 1 or more and 5 or less, and further more preferably 1 or more and 3 or less amino acids are deleted, inserted, substituted or added. In the present specification, examples of the "nucleotide sequence wherein one or several nucleotides are deleted, inserted, substituted or added" include nucleotide sequences wherein preferably 1 or more and 30 or less, more preferably 1 or more and 15 or less, and further more preferably 1 or more and 10 or less nucleotides are deleted, inserted, substituted or added. As used herein, the "addition" of amino acids or nucleotides includes addition of one or several amino acids or nucleotides to one terminus or both termini of the sequence.

As used herein, the "corresponding position" on the amino acid sequence or the nucleotide sequence can be determined by aligning a target sequence and a reference sequence (for example, the amino acid sequence of SEQ ID NO: 1) so as to give the maximum homology. The alignment of amino acid sequences or nucleotide sequences can be carried out by using a known algorithm, and the procedure thereof is known to those skilled in the art. For example, the alignment can be carried out by using the Clustal W multiple alignment program (Thompson, J. D. et al., 1994, Nucleic Acids Res. 22: 4673-4680) in a default setting. Alternatively, Clustal W2 and Clustal omega which are modified versions of Clustal W can be used. Clustal W, Clustal W2 and Clustal omega can be used on, for example, the website of Clustal run by University College Dublin [www.clustal.org], the website of European Bioinformatics Institute: EBI [www.ebi.ac.uk/index.html], or the website of DNA Data Bank of Japan run by National Institute of Genetics (DDBJ [www.ddbj.nig.ac.jp]). The position of a target sequence aligned at an arbitrary position of a reference sequence by the alignment is considered as a "position corresponding to" the arbitrary position.

Those skilled in the art can further make a minor adjustment so that the alignment of the amino acid sequence obtained as described above will be optimized. Such an optimum alignment is preferably determined by considering an analogy between amino acid sequences, a frequency of gaps inserted, and the like. Here, the analogy between amino acid sequences refers to a ratio (%) of the number of positions, at which, when two amino acid sequences are aligned, the two sequences have identical or analogous amino acid residues, to the number of amino acid residues over the full length. The term "analogous amino acid residues" means amino acid residues which are among the 20 proteinogenic amino acids, have similar properties in terms of polarity and electric charge, and undergo so-called conservative substitution. Groups of such analogous amino acid residues are well known to those skilled in the art, and examples thereof include, but are not limited to, arginine and lysine or glutamine; glutamic acid and aspartic acid or glutamine; serine and threonine or alanine; glutamine and asparagine or arginine; and leucine and isoleucine respectively.

The position of an amino acid residue of a target amino acid sequence aligned at a position corresponding to an arbitrary position of a reference sequence by the alignment is considered as a "position corresponding to" the arbitrary position, and the amino acid residue is referred to as an "amino acid residue at an corresponding position".

As used herein, the term "parent" polypeptide of a mutant polypeptide" refers to a polypeptide which is turned into the mutant polypeptide by application of a predetermined mutation to an amino acid residue of the parent polypeptide. In other words, the "parent" polypeptide is a polypeptide before adding mutation for forming a polypeptide mutant. Similarly, the term ""parent" polynucleotide of a mutant polynucleotide" refers to a polynucleotide which is turned into the mutant polynucleotide by application of a predetermined mutation to a nucleotide of the parent polynucleotide. In other words, the "parent" polynucleotide is a polynucleotide before adding mutation for forming a mutant polynucleotide.

As used herein, the term "operably connecting" a control region such as a promoter and a gene means that the gene and the control region are connected so that the gene can be expressed under the control of the control region. The procedure of "operably connecting" a gene and a control region is well known to those skilled in the art.

As used herein, the terms "upstream" and "downstream" related to a gene refer, respectively, to the upstream and the downstream of the gene in a transcription direction. For example, the terms "gene disposed downstream of a promoter" means that the gene is present on the 3' side of the promoter in a DNA sense strand, and the term "upstream of a gene" means a region on the 5' side of the gene in the DNA sense strand.

As used herein, the term "intrinsic" which is used for functions, properties and characteristics of cells is intended to mean that the functions, properties and characteristics originally exist in the cells. In contrast, the term "foreign" is intended to mean that the functions, properties and characteristics do not originally exist in the cells, but are introduced from the outside. For example, the term "foreign" gene or polynucleotide" refers to a gene or polynucleotide introduced into cells from the outside. The foreign gene or polynucleotide may be derived from an organism of the same type as cells into which it is introduced, or an organism different in type from the cells (i.e., a heterologous gene or polynucleotide).

The present invention relates to providing a mutant protease having improved cleaning performance against protein stains in a high-concentration detergent liquid.

For removing tough stains such as smears, a stock solution of a liquid detergent may be directly applied to the stains without being diluted with water. For stains containing proteins, such as spilled foods and stains on collars and sleeves, application of a liquid detergent containing a protease may be effective. However, the stock solution of a liquid detergent has a high concentration of cleaning components such as a surfactant, and such a condition is severe to proteins. It is desired to improve the cleaning performance of the protease in a solution containing cleaning components at a high concentration, such as a stock solution of a liquid detergent.

The present inventors have found that a mutant protease derived from protease KP43 (see Patent Literature 1) having a molecular weight of 43 000 has improved cleaning performance against protein stains even in a stock solution of a liquid detergent (hereinafter, referred to simply as cleaning performance).

The mutant protease of the present invention has improved cleaning performance against protein stains in a high-concentration detergent liquid (for example, a stock solution of a liquid detergent). The mutant protease of the present invention is effective as a protease used for cleaning with a high-concentration detergent liquid, such as application cleaning or immersion cleaning. For example, the mutant protease of the present invention is effective as a protease which is blended in a detergent used for cleaning stains by bringing a stock solution of liquid detergent or a high-concentration detergent liquid into direct contact with the stains.

The mutant protease of the present invention has at least one mutation selected from the group consisting of the following mutations (A) to (G) relative to a parent protease consisting of the amino acid sequence of SEQ ID NO: 1 or an amino acid sequence having an identity of at least 90% with SEQ ID NO: 1:

(A) substitution of Arg or Lys, preferably Arg, for an amino acid residue at position 191 of SEQ ID NO: 1 or a position corresponding thereto;
(B) substitution of Arg or Lys, preferably Arg, for an amino acid residue at position 17 of SEQ ID NO: 1 or a position corresponding thereto;
(C) substitution of Ala or Phe, preferably Ala, for an amino acid residue at position 141 of SEQ ID NO: 1 or a position corresponding thereto;
(D) substitution of Arg or Lys, preferably Lys, for an amino acid residue at position 243 of SEQ ID NO: 1 or a position corresponding thereto;
(E) substitution of Arg or Lys, preferably Lys, for an amino acid residue at position 300 of SEQ ID NO: 1 or a position corresponding thereto;
(F) substitution of Arg or Lys, preferably Arg, for an amino acid residue at position 302 of SEQ ID NO: 1 or a position corresponding thereto; and
(G) substitution of Arg or Lys, preferably Arg, for an amino acid residue at position 311 of SEQ ID NO: 1 or a position corresponding thereto.

Preferably, the mutant protease of the present invention has the mutation (A) relative to the parent protease. As a preferred embodiment, the mutant protease of the present invention has the mutation (A) and at least one selected from the group consisting of the mutations (B) to (G), relative to the parent protease.

As one embodiment, the mutant protease of the present invention has the mutation (A) and at least one mutation selected from the group consisting of the mutations (B) and (D), relative to the parent protease. As another embodiment, the mutant protease of the present invention has the mutation (A) and any one selected from the group consisting of the mutations (B) and (D). Preferably, the mutation (A) is substitution of Arg for an amino acid residue at position 191 or a position corresponding thereto, the mutation (B) is substitution of Arg for an amino acid residue at position 17 or a position corresponding thereto, and the mutation (D) is substitution of Lys for an amino acid residue at position 243 or a position corresponding thereto.

As one embodiment, the mutant protease of the present invention has, in addition to at least one selected from the group consisting of the mutations (A) to (G), the following mutation (H):

(H) substitution of Gln for an amino acid residue at position 82 of SEQ ID NO: 1 or a position corresponding thereto.

Having the mutation (H) in addition to any of the mutations (A) to (G) leads to further improvement of the cleaning performance of the mutant protease of the present invention in a high-concentration detergent liquid. As a preferred embodiment, the mutant protease of the present invention has the mutation (A) and the mutation (H). Preferably, the mutation (A) is substitution of Arg for an amino acid residue at position 191 or a position corresponding thereto.

As one embodiment, the mutant protease of the present invention may have at least one selected from the group consisting of the mutations (B) to (G). As a preferred embodiment, the mutant protease of the present invention has the mutation (E) and the mutation (F) relative to the parent protease. Having the mutations (E) and (F) leads to further improvement of the cleaning performance of the mutant protease of the present invention in a high-concentration detergent liquid. Preferably, the mutation (E) is substitution of Lys for an amino acid residue at position 300 or a position corresponding thereto, and the mutation (F) is substitution of Arg for an amino acid residue at position 302 or a position corresponding thereto.

Examples of the parent protease of the mutant protease of the present invention include a protease consisting of the amino acid sequence of SEQ ID NO: 1, and a protease consisting of an amino acid sequence having an identity of at least 90% with the amino acid sequence of SEQ ID NO: 1.

Preferred examples of the protease consisting of an amino acid sequence having an identity of at least 90% with the amino acid sequence of SEQ ID NO: 1 include a protease having an identity of 95% or more, more preferably an identity of 96% or more, further more preferably an identity of 97% or more, further more preferably an identity of 98% or more, and further more preferably an identity of 99% or more with the amino acid sequence of SEQ ID NO: 1. Other examples of the protease consisting of an amino acid sequence having an identity of at least 90% with the amino acid sequence of SEQ ID NO: 1 include a protease consisting of an amino acid sequence wherein one or several amino acids are deleted, inserted, substituted or added relative to the amino acid sequence of SEQ ID NO: 1. The protease which consists of an amino acid sequence having an identity of at least 90% with the amino acid sequence of SEQ ID NO: 1, and is used as a parent protease in the present invention may be a mutant protease derived from a protease consisting of the amino acid sequence of SEQ ID NO: 1.

Preferably, in the amino acid sequence of the parent protease, the amino acid residue at a position corresponding to position 30 of SEQ ID NO: 1 is aspartic acid, the amino acid residue at a position corresponding to position 68 is histidine, and the amino acid residue at a position corresponding to position 255 is serine. More preferably, the parent protease has, at positions corresponding, respectively, to positions of SEQ ID NO: 1 which are shown as (i) in Table 1 below, amino acid residues shown as (ii) in Table 1. The amino acid residues shown in Table 1 are highly conserved amino acid residues between protease KP43 which is the origin of the parent protease (Patent Literature 1) and a mutant protease derived therefrom (Saeki et al, Journal of bioscience and Bioengineering, 2007, 103: 501-508).

[Table 1]

| (i) Position | (ii) Amino acid residue | (i) Position | (ii) Amino acid residue |
|---|---|---|---|
| 10 | Alanine | 159 | Alanine |
| 18 | Glycine | 161 | Glycine |
| 21 | Glycine | 162 | Asparagine |
| 26 | Valine | 173 | Proline |
| 27 | Alanine | 182 | Valine |
| 28 | Valine | 183 | Glycine |

(continued)

| (i) Position | (ii) Amino acid residue | (i) Position | (ii) Amino acid residue |
|---|---|---|---|
| 30 | Aspartic acid | 184 | Alanine |
| 32 | Glycine | 203 | Alanine |
| 43 | Histidine | 205 | Phenylalanine |
| 52 | Alanine | 206 | Serine |
| 64 | Aspartic acid | 209 | Glycine |
| 66 | Asparagine | 222 | Alanine |
| 68 | Histidine | 223 | Proline |
| 69 | Glycine | 224 | Glycine |
| 70 | Threonine | 225 | Threonine |
| 71 | Histidine | 229 | Serine |
| 72 | Valine | 253 | Glycine |
| 73 | Alanine | 254 | Threonine |
| 74 | Glycine | 255 | Serine |
| 85 | Glycine | 256 | Methionine |
| 87 | Alanine | 257 | Alanine |
| 88 | Proline | 259 | Proline |
| 92 | Leucine | 261 | Valine |
| 106 | Glycine | 262 | Alanine |
| 118 | Alanine | 263 | Glycine |
| 129 | Serine | 266 | Alanine |
| 131 | Glycine | 289 | Leucine |
| 145 | Valine | 297 | Glycine |

The parent protease of the mutant of the present invention is a polypeptide having proteolytic activity on the alkali side (preferably at a pH of 8 or more). Preferably, the parent protease is a polypeptide having the optimum pH on the alkali side (preferably at a pH of 8 or more). More preferably, the parent protease has any of the following enzymological properties of a protease consisting of the amino acid sequence of SEQ ID NO: 1 (see Patent Literature 1). 1) The protease has oxidant resistance, acts on the alkali side (at a pH of 8 or more), and is stable. Here, the phrase "having oxidant resistance" means that the residual activity (synthetic substrate method) of the protease is at least 50% or more after left to stand in a 50 mM solution of hydrogen peroxide (containing 5 mM calcium chloride) (20 mM Britton-Robinson buffer solution, pH 10) at 20°C for 20 minutes. 2) The protease exhibits a residual activity of 80% or more when treated at 50°C and pH 10 for 10 minutes. 3) Activity is impaired by diisopropyl fluorophosphate (DFP) and phenylmethane sulfonyl fluoride (PMSF). 4) The molecular weight by SDS-PAGE is 43 000 ± 2 000. More preferably, the parent protease has all the enzymological properties 1) to 4).

The mutant protease of the present invention can be produced by applying at least one mutation selected from the group consisting of the mutations (A) to (G) in the amino acid sequence of SEQ ID NO: 1 to the amino acid sequence of the parent protease. Preferably, the mutant protease of the present invention can be produced by applying, in addition to at least one mutation selected from the group consisting of the mutations (A) to (G), the mutation (H) to the amino acid sequence of the parent protease.

Accordingly, the mutant protease of the present invention has at least one amino acid residue selected from the group consisting of the following amino acid residues (a) to (g):

(a) Arg or Lys, preferably Arg, at a position corresponding to position 191 of SEQ ID NO: 1;
(b) Arg or Lys, preferably Arg, at a position corresponding to position 17 of SEQ ID NO: 1;
(c) Ala or Phe, preferably Ala, at a position corresponding to position 141 of SEQ ID NO: 1;
(d) Arg or Lys, preferably Lys, at a position corresponding to position 243 of SEQ ID NO: 1;

(e) Arg or Lys, preferably Lys, at a position corresponding to position 300 of SEQ ID NO: 1;

(f) Arg or Lys, preferably Arg, at a position corresponding to position 302 of SEQ ID NO: 1; and

(g) Arg or Lys, preferably Arg, at a position corresponding to position 311 of SEQ ID NO: 1.

Preferably, the mutant protease of the present invention has a sequence identity of at least 90% with the amino acid sequence of SEQ ID NO: 1.

Preferably, the mutant protease of the present invention has the amino acid residue (a). In a preferred embodiment, the mutant protease of the present invention has the amino acid residue (a), and at least one amino acid residue selected from the group consisting of the amino acid residues (b) to (g).

In an embodiment, the mutant protease of the present invention has the amino acid residue (a), and at least one amino acid residue selected from the group consisting of the amino acid residues (b) and (d). In another embodiment, the mutant protease of the present invention has the amino acid residue (a), and any one of the amino acid residues (b) and (d). Preferably, the amino acid residue (a) is Arg, the amino acid residue (b) is Arg, and the amino acid residue (d) is Lys.

In an embodiment, the mutant protease of the present invention has, in addition to at least one selected from the group consisting of the amino acid residues (a) to (g), the following amino acid residue (h):

(h) Gln at a position corresponding to position 82 of SEQ ID NO: 1.

In a preferred embodiment, the mutant protease of the present invention has the amino acid residues (a) and (h). Preferably, the amino acid residue (a) is Arg.

In an embodiment, the mutant protease of the present invention may have at least one amino acid residue selected from the group consisting of the amino acid residues (b) to (g). In a preferred embodiment, the mutant protease of the present invention has the amino acid residues (e) and (f). Preferably, the amino acid residue (e) is Lys, and the amino acid residue (f) is Arg.

The mutant protease of the present invention has improved cleaning performance against protein stains in a detergent liquid containing a surfactant at a high concentration (a so-called high-concentration detergent liquid). More specifically, the mutant protease of the present invention can exhibit higher cleaning performance in a high-concentration detergent liquid than a protease consisting of the amino acid sequence of SEQ ID NO: 1. Preferably, the mutant protease of the present invention can exhibit higher cleaning performance in a stock solution of concentrated liquid detergent than a protease consisting of the amino acid sequence of SEQ ID NO: 1. Preferably, the mutant protease of the present invention can exhibit cleaning performance at least corresponding to or higher than that of a protease consisting of the amino acid sequence of SEQ ID NO: 1, in a detergent liquid obtained by dilution with water to a degree that it is used in normal cleaning (a so-called normal detergent liquid). Therefore, the mutant protease of the present invention can be used as an enzyme for detergent, in particular, an enzyme for detergent which is used in cleaning with a high-concentration detergent liquid (for example, application cleaning or immersion cleaning). For example, the mutant protease of the present invention is effective as a protease which is blended in a concentrated liquid detergent used for cleaning of stains by directly applying a stock solution of the detergent or a high-concentration detergent liquid to the stains. For example, the mutant protease of the present invention is effective as a protease which is blended in a concentrated liquid detergent used for cleaning of stains by immersing the stains in a stock solution of the detergent or a high-concentration detergent liquid.

Examples of the "protein stains" as used herein include stains from food containing protein, and body-derived components containing protein (for example, blood, urine, scurf, sweat, sebum, and the like). More specific examples of the "protein stains" include spilled food on clothing, and stains on collars and sleeves.

The "detergent liquid" as used herein includes a stock solution of liquid detergent, and detergent aqueous solutions obtained by diluting a liquid detergent or a solid detergent (for example, a powder detergent) with water. As used herein, the term "high-concentration detergent liquid" refers to a detergent liquid containing a surfactant at a high concentration, preferably at 10 mass% or more, more preferably at 20 mass% or more, for example, from 10 to 90 mass%, preferably from 20 to 90 mass%, and more preferably from 20 to 75 mass%. Examples of the high-concentration detergent liquid include stock solutions of concentrated liquid detergent, or aqueous solutions containing the concentrated liquid detergent at a high concentration. As used herein, the term "concentrated liquid detergent" refers to a liquid detergent containing a surfactant at 40 mass% or more, preferably from 40 to 90 mass%, more preferably from 45 to 90 mass%, and further more preferably from 50 to 75 mass%. Examples of the "concentrated liquid detergent" include liquid detergents for clothes with a label indicating that the standard amount of use with 30 L of water in a water tank laundry machine is from 7 to 13 g or less.

As used herein, the term "cleaning with a high-concentration detergent liquid" refers to a cleaning method wherein a high-concentration detergent liquid is brought into direct contact with stains on an object to be cleaned (for example, a cloth, or a hard surface of an eating utensil, cookware or the like), and is left to stand or engaged in cleaning for a predetermined time as necessary, and the object to be cleaned is then washed with water. Examples of the "cleaning with a high-concentration detergent liquid" include application cleaning, and immersion cleaning. The term "application cleaning" refers to a cleaning method wherein a high-concentration detergent liquid is directly applied to stains on an object to be cleaned, and is left to stand or engaged in cleaning for a predetermined time as necessary, and the object to be cleaned is then washed with water. The term "immersion cleaning" refers to a cleaning method wherein stains on an object to be cleaned are immersed in a high-concentration detergent liquid, and left to stand or cleaned with the high-concentration detergent liquid for a predetermined time as necessary, and the object to be cleaned is then washed with water. Thus, for the cleaning with the high-concentration detergent liquid, examples of the approach for bringing the high-concentration detergent liquid into direct contact with stains on an object to be cleaned include application of the high-concentration detergent liquid to the stains (by, for example, rolling on, spraying or dropping), and immersion of the stains in the high-concentration detergent liquid.

The mutant protease of the present invention may have, in addition to at least one selected from the group consisting of the mutations (A) to (G), mutations (for example, deletion, substitution, addition or insertion) at other arbitrary positions relative to the parent protease so long as the cleaning performance higher than the parent protease can be retained in the high-concentration detergent liquid as described above. The mutations may naturally occur, or may be artificially introduced. However, the mutant protease of the present invention is preferably identical to the parent protease in amino acid residues at least at positions 30, 68 and 255 of SEQ ID NO: 1. More preferably, the mutant protease of the present invention is identical to the parent protease in amino acid residues at positions corresponding, respectively, to positions of SEQ ID NO: 1 which are shown as (i) in Table 1.

In the present invention, various mutagenesis techniques known in the art can be used as approaches for mutating the amino acid residues of the parent protease. For example, in a polynucleotide encoding the amino acid sequence of the parent protease (hereinafter, also referred to as a parent polynucleotide), a nucleotide sequence encoding an amino acid residue to be mutated is mutated into a nucleotide sequence encoding an amino acid residue after the mutation, and a protein is expressed from the mutant polynucleotide, thereby enabling the obtainment of an intended mutant protease.

An intended mutation can be introduced into a parent polynucleotide by various site-directed mutagenesis methods well known to those skilled in the art, basically on the basis of, for example, PCR amplification using the parent polynucleotide as template DNA and replication reactions of various DNA polymerases. The site-directed mutagenesis method can be carried out through an arbitrary method such as an inverse PCR method or an annealing method (Matsumura et al, ed., "Shin Idenshi Kogaku Handbook, the revised fourth edition", Yodosha Co., Ltd., p. 82-88). It is also possible to use various commercially available site-directed mutagenesis kits such as QuickChange II Site-Directed Mutagenesis Kit and QuickChange Multi Site-Directed Mutagenesis Kit from Stratagene Corporation.

The site-directed mutagenesis in a parent polynucleotide can be most typically carried out by using a mutation primer containing a nucleotide mutation to be introduced. The mutation primer may be designed so as to contain a nucleotide sequence which is annealed to a region containing a nucleotide sequence encoding an amino acid residue to be mutated in a parent polynucleotide and which has, instead of the nucleotide sequence (codon) encoding the amino acid residue to be mutated, a nucleotide sequence having a nucleotide sequence (codon) encoding an amino acid residue after the mutation. The nucleotide sequence (codon) encoding the amino acid residue before and after the mutation can be recognized and selected appropriately on the basis of ordinary textbooks or the like by those skilled in the art. Alternatively, for the site-directed mutagenesis, a method can be used in which DNA fragments obtained by amplifying the upstream side and the downstream side of the mutant site respectively, using separately two complementary primers containing a nucleotide mutation to be introduced are connected together by SOE (splicing by overlap extension)-PCR (Horton et al, Gene, 1989, 77(1): p. 61-68).

DNA containing a parent polynucleotide can be prepared by chemically synthesizing a corresponding nucleotide sequence on the basis of the amino acid sequence of a parent protease. Alternatively, DNA containing a parent polynucleotide can be extracted by a known approach from a microbial strain holding the DNA. A mutation primer can be produced by a well-known oligonucleotide synthesis method such as a phosphoramidite (Nucleic Acids Research, 1989, 17: 7059-7071). Such primer synthesis can also be carried out by using, for example, a commercially available oligonucleotide synthesis apparatus (e.g., manufactured by ABI Inc.). The above-described site-directed mutagenesis is carried out with a primer set containing the mutation primer, and a parent polynucleotide as template DNA, whereby a polynucleotide which encodes a mutant protease and which has an intended mutation introduced can be obtained.

Accordingly, the present invention also provides a mutant protease gene. The mutant protease gene of the present invention is a polynucleotide encoding the mutant protease of the present invention. The polynucleotide of the present invention can contain single-stranded or double-stranded DNA, cDNA, RNA, or other artificial nucleic acids. The DNA, cDNA and RNA may be chemically synthesized. The polynucleotide of the present invention may contain the nucleotide sequence of an untranslated region (UTR) in addition to an open reading frame (ORF).

The present invention also provides a vector containing a polynucleotide encoding the mutant protease of the present invention. The vector can be produced by inserting the polynucleotide of the present invention into an arbitrary vector, followed by connection, by a conventional method. The type of the vector is not limited, and may be an arbitrary vector such as a plasmid, a phage, a phagemid, a cosmid, a virus, a YAG vector or a shuttle vector. The vector is preferably a vector capable of amplifying in bacteria, in particular, *Bacillus* bacteria, and more preferably an expression vector capable of inducing the expression of an induced gene in *Bacillus* bacteria, but is not limited thereto. Among them, a shuttle vector which is capable of replicating in either *Bacillus* bacteria or other organisms can be suitably used for the recombinant production of the mutant protease of the present invention. Examples of the preferred vector include, but are not limited to, shuttle vectors such as pHA3040SP64 (JP-A-2013-233141), pHSP64R or pASP64 (JP-B-3492935), pHA64, pHY300PLK (an expression vector capable of transforming both *Escherichia coli* and *Bacillus subtilis;* Ishikawa and Shibahara, Jpn J Genet, 1985, 60: 235-243), and pAC3 (Moriyama et al, Nucleic Acids Res, 1988, 16: 8732); plasmids which can be used in transformation of *Bacillus* bacteria, such as pUB110 (Gryczan et al, J Bacteriol, 1978, 134: 318-329), and pTA10607 (Bron et al, Plasmid, 1987, 18: 8-15); and secretion vectors capable of imparting a secretion signal to a recombinant protein (Yamane et al., "Formation of Fused Extracellular Thermostable α-amylase Using Bacillus subtilis Secretion Vector", Journal of the Japanese Society of Starch Science, 34 (1987), 163-170). It is also possible to use plasmids derived from *Escherichia coli* (for example, pET22b (+), pBR322, pBR325, pUC118, pUC119, pUC18, pUC19, pBluescript, and the like).

In the case of the recombinant production of the mutant protease of the present invention, the vector is preferably an expression vector. The expression vector may contain useful sequences such as various elements absolutely necessary for expression in host organisms, such as a transcription promoter, a terminator and a ribosome binding site; cis-elements such as a poly linker and an enhancer; poly A addition signals; ribosome binding sequences (SD sequences); and selection marker genes such as genes resistant to drugs (for example, ampicillin, neomycin, kanamycin, tetracycline and chloramphenicol) as a case requires.

The present invention also provides a transformant containing a polynucleotide encoding the mutant protease of the present invention or a vector containing the polynucleotide. The transformant can be produced by introducing a polynucleotide encoding the mutant protease of the present invention or a vector containing the polynucleotide (preferably, a recombinant expression vector) into a host. Therefore, the transformant contains a foreign polynucleotide encoding the mutant protease of the present invention.

Examples of the host of the transformant include arbitrary cells such as microorganisms including bacteria such as *Escherichia coli* and *Bacillus subtilis,* and yeast cells, and insect cells, animal cells (for example, mammal cells), and plant cells. The host is preferably *Bacillus* bacteria, and more preferably *Bacillus subtilis* or a mutant strain thereof. Therefore, the transformant of the present invention is preferably recombinant *Bacillus* bacteria, and more preferably recombinants of *Bacillus subtilis* or a mutant strain thereof.

For the transformation of the host, a well-known transformation technique such as a calcium phosphate method, an electroporation method, a lipofection method, a particle gun method or PEG method can be applied. Examples of the transformation method which can be applied to *Bacillus* bacteria include a competent cell transformation method (J Bacteriol, 1967, 93: 1925-1937), an electroporation method (FEMS Microbiol Lett, 1990, 55: 135-138), a protoplast transformation method (Mol Gen Genet, 1979, 168: 111-115), and a Tris-PEG method (J Bacteriol, 1983, 156: 1130-1134).

The mutant protease of the present invention can be produced by culturing the transformant of the present invention. Accordingly, the present invention also provides a method for producing a mutant protease using the transformant of the present invention. The culture of the transformant for producing a mutant protease can be carried out by a common method for those skilled in the art. For example, as the culture medium used for culturing a transformant based on a microbial host such as *Escherichia coli* and yeast cells any medium may be used so long as it contains carbon sources, nitrogen sources, inorganic salts that the microbial host can assimilate, and the like, and enables the transformant to be cultured with efficiency. The culture medium may be either a natural culture medium or a synthetic culture medium. For example, for culture of a *Bacillus subtilis* transformant for producing a recombinant protein, LB culture medium, 2XYT culture medium, 2XL-maltose culture medium, CSL fermentation culture medium or the like can be used. To the culture medium, a drug relevant to the type of a drug-resistant gene (selection marker gene) introduced into the transformant may be added. When microorganisms transformed with an expression vector based on an inducible promoter are cultured, an inducer may be added to the culture medium as necessary. For example, isopropyl-1-thio-β-D-galactoside (IPTG) and the like can be added to the culture medium when microorganisms transformed with an expression vector based on a Lac promoter are cultured, and indoleacetic acid (IAA) and the like can be added to the culture medium when microorganisms transformed with an expression vector based on a trp promoter are cultured.

Alternatively, using a cell-free translation system, the mutant protease of the present invention may be expressed from a polynucleotide encoding the mutant protease of the present invention, or a transcription product thereof. The term "cell-free translation system" means that an *in vitro* transcriptional translation system or an *in vitro* translation system is formed by adding a reagent necessary for translation of protein, such as an amino acid, to a suspension obtained by mechanically destroying cells as a host.

The mutant protease of the present invention produced by using the transformant or in the cell-free translation system can be separated or purified from a culture solution, a cell homogenate, a reaction liquid of a cell-free translation system or the like by using common methods used for the protein purification, for example, centrifugation, ammonium sulfate precipitation, gel chromatography, ion-exchange chromatography and affinity chromatography, alone or in combination appropriately. Alternatively, a solution such as a culture supernatant or a lysis solution supernatant separated or concentrated by means of centrifugation, an ultrafiltration filter or the like can be directly used as a crude enzyme liquid. If the expressed mutant protease is not secreted from the inside of cells, the cells may be disrupted, followed by separation and purification of protein.

The procedures of DNA extraction, preparation of mRNA, production of cDNA, PCR, RT-PCR, production of a library, ligation into a vector, transformation of cells, determination of a nucleotide sequence of DNA, chemical synthesis of a nucleic acid, determination of an amino acid sequence on the N terminus side of protein, mutagenesis, extraction of protein and the like, which are employed in the present invention, can be carried out by methods described in usual written experiment guides. Examples of the written experiment guide include Sambrook et al., Molecular Cloning, A laboratory manual, 2001, 3rd Ed., Sambrook, J & Russell, DW. Cold Spring Harbor Laboratory Press. Further, for experiments on genetic recombination in *Bacillus subtilis,* it is possible to refer to general written experiment guides on genetic manipulation in *Bacillus subtilis,* such as "7.2 Kosokin Kei" "Zoku Seikagaku Zikken Koza 1. Idenshi Kenkyu Ho II", written by Hirohumi Yoshikawa, 1986, Tokyo Kagaku Dojin (Tokyo), p. 150-169.

A mutant protease obtained by the production method of the present invention has improved cleaning performance in a high-concentration detergent liquid as compared to a parent protease, and is useful as a protease which is used for a detergent used for cleaning with a high-concentration detergent liquid (for example, application cleaning or immersion cleaning). Accordingly, another aspect of the present invention can be a method for improving the cleaning performance of a protease against protein stains in a high-concentration detergent liquid, containing applying at least one selected from the group consisting of the mutations (A) to (G) to the amino acid sequence of a parent protease as described above.

The mutant protease of the present invention is useful as an enzyme for detergent, and more suitable as, in particular, an enzyme which is blended in a detergent used for cleaning with a high-concentration detergent liquid (for example, application cleaning or immersion cleaning). Accordingly, the present invention also provides a detergent composition containing the mutant protease of the present invention. The detergent composition may be a solid (for example, powder) detergent composition, but is preferably a liquid detergent composition, and more preferably a concentrated liquid detergent. The detergent composition is preferably a detergent composition for application cleaning or immersion cleaning, and more preferably a detergent composition for application cleaning.

The content of the mutant protease of the present invention in the detergent composition of the present invention is not particularly restricted so long as it is an amount which allows the protease to exhibit activity. The content of the mutant protease per kg of the detergent composition is preferably from 0.1 to 25 000 U, more preferably from 0.1 to 5 000 U, and further more preferably from 0.1 to 2 500 U. The activity of the protease (U) in the present specification is measured by the following method: 0.9 mL of a 1/15 M phosphate buffer solution (for example, pH 7.4, a solution obtained by dissolving one packet of Phosphate Buffer Powder (167-14491) from Wako Pure Chemical Industries, Ltd. (containing 7.6 g of $Na_2HPO_4$ (anhydrous) and 1.8 g of $KH_2PO_4$ (anhydrous) per packet) in 1 L of ion-exchange water) and 0.05 mL of a 40 mM Glt-Ala-Ala-Pro-Leu-p-nitroanilide/dimethylsulfoxide solution are put in a test tube, and maintained at 30°C for 5 minutes. 0.05 mL of an enzyme solution is added thereto, the reaction is carried out at 30°C for 10 minutes, 2.0 mL of a 5% (w/v) citric acid aqueous solution is then added to terminate the reaction, and the absorbance at 420 nm is measured by a spectrophotometer. Here, one unit of the enzyme (U) is an amount which allows generation of 1 μmol of p-nitroaniline per minute in the reaction.

The detergent composition of the present invention contains a surfactant and water in addition to the mutant protease of the present invention. As the surfactant, arbitrary surfactants such as a nonionic surfactant, an anionic surfactant, a cationic surfactant and an ampholytic surfactant can be used singly or in combinations of two or more thereof. The content of the surfactant in the detergent composition of the present invention is preferably from 10 to 90 mass%, more preferably from 10 to 80 mass%, and further more preferably from 30 to 75 mass%. When the detergent composition of the present invention is a concentrated liquid detergent, the content of the surfactant in the detergent composition is preferably from 40 to 90 mass%, more preferably from 45 to 90 mass%, and further more preferably from 50 to 75 mass%.

As the nonionic surfactant, any nonionic surfactant may be used so long as it has a C8-C22 hydrocarbon group which is generally blended in liquid detergent, with C2 oxyalkylene groups added in an amount of no less than several moles. Examples thereof include:

$R_1$-(AO)m-H ($R_1$ = C8-C22 hydrocarbon, AO = C2-C5 oxyalkylene group, m = from 16 to 35) [JP-A-2010-275468];
$R_1$O-(EO)1-(AO)m-(EO)n-H ($R_1$ = C8-C18 hydrocarbon, EO = C2 oxyalkylene group, AO = C3-C5 oxyalkylene group, 1 = from 3 to 30, m = from 1 to 5, 1 + n = from 14 to 50) [JP-A-2010-265445, JP-A-2011-63784];
$R_1$O-(EO)m/(AO)n-H ($R_1$ = C8-C22 hydrocarbon, EO = C2 oxyalkylene group, AO = C3-C5 oxyalkylene group, m = from 10 to 30, n = from 0 to 5, EO and AO are bonded randomly or with regularity) [JP-A-2010-189551];
$R_l$(CO)lO-(EO)m/(AO)n-$R_2$ ($R_1$ = C8-C22 hydrocarbon, EO = C2 oxyalkylene group, AO = C3-C5 oxyalkylene group, 1 = from 0 to 1, m = from 14 to 50, n = from 1 to 5, $R_2$ = hydrogen (1 = 0) or C1-C3 alkyl group, EO and AO are bonded randomly or with regularity) [JP-A-2010-229385];
$R_1$O-(EO)m-(AO)n-H ($R_1$ = C8-C22 hydrocarbon, EO = C2 oxyalkylene group, AO = C3-C5 oxyalkylene group, m = from 15 to 30, n = from 1 to 5) [JP-A-2010-229387];

R$_1$O-(AO)m/ Gly)n-H and/or R$_2$-COO-(AO)p/(Gly)q-H (R$_1$ = C8-C22 hydrocarbon group, R$_2$ = C7-C21 hydrocarbon group, AO = C2-C3 oxyalkylene group, Gly = glycerol group, m = from 0 to 5, n = from 2 to 10, p = from 0 to 5, q = from 2 to 10, AO and Gly are bonded randomly or with regularity) [JP-A-2010-254881];

R$_1$-COO-(PO)m/(EO)n-R$_2$ (R$_1$ = C7-C21 hydrocarbon, COO = carbonyloxy group, R$_2$ = C1-C3 alkyl group, PO = oxypropylene group, EO = oxyethylene group, m = from 0.3 to 5, n = from 8 to 25, PO and EO are bonded randomly or with regularity) [JP-A-2010-265333];

R$_1$O-(EO)1-(PO)m-(EO)n-H (R$_1$ = C8-C20 hydrocarbon, EO = C2 oxyalkylene group, PO = oxypropylene group, 1> = 1, n> = 1, 0<m<1+n, EO and PO are bonded with regularity) [WO 98/24865];

R$_1$O-(EO)m-(PO)n-H (R$_1$ = C10-C16 alkyl group or alkenyl group, EO = ethylene oxide group, PO = propylene oxide group, m = from 5 to 15, n = from 1 to 3) [JP-A-1996-157867];

R$_1$(CO)-(EO)m-OR$_2$ (R$_1$ = linear or branched C11-C13 alkyl group or alkenyl group, R$_2$ = C1-C3 alkyl group, EO = ethylene oxide group, m = from 10 to 20) [JP-A-2008-7706, JP-A-2009-7451, JP-A-2009-155594 and JP-A-2009-155606] ;

R$_1$(CO)-(AO)m-OR$_2$ (R$_1$ = linear or branched C9-C13 alkyl group or alkenyl group, AO = C2-C4 oxyalkylene group, R$_2$ = C1-C3 alkyl group, m = from 5 to 30) [JP-A-2009-144002, JP-A-2009-173858, JP-A-2010-189612]; and fatty acid alkanolamide, fatty acid alkanolglucamide and alkyl polyglycoside.

Examples of the anionic surfactant include carboxylate-type anionic surfactants, sulfonic acid-type or sulfuric acid ester-type anionic surfactants, non-soap-based anionic surfactants, linear alkyl benzenesulfonic acids, benzenesulfonic acid or salts thereof, polyoxybenzenesulfonic acid or salts thereof, polyoxyethylene alkyl sulfuric acid ester salts, polyoxyalkylene alkyl ether sulfuric acid ester salts, α-olefin sulfonic acid salts, alkyl benzenesulfonic acid salts, α-sulfo fatty acid salts, fatty acid soaps, phosphoric acid ester salt-based surfactants, acyl alaninate, acyl taurate, alkyl ether carboxylate, and alcohol sulfuric acid esters.

Examples of the cationic surfactant include quaternary ammonium salts having a long-chain alkyl group, tertiary amines having one long-chain alkyl group, alkyltrimethylammonium salts, dialkyldimethylammonium salts, and alkylpyridinium salts. Quaternary ammonium-type surfactants having one long-chain alkyl group having 8 to 22 carbon atoms, and tertiary amines having a long-chain alkyl group having 8 to 22 carbon atoms are preferable.

Examples of a zwitterionic surfactant include ampholytic surfactants of alkyl betaine type, alkylamide betaine type, imidazoline type, alkyl aminosulfone type, alkyl aminocarboxylic acid type, alkylamide carboxylic acid type, amide amino acid type or phosphoric acid type, such as alkyl acetic acid betaine, alkanolamide propyl acetic acid betaine, alkyl imidazolines and alkyl alanines. Sulfobetaine or carbobetaine having an alkyl group having 10 to 18 carbon atoms is preferable.

The detergent composition of the present invention may further contain components which are typically used for detergent compositions, for example, a water-soluble polymer, a water-miscible organic solvent, an alkali agent, an organic acid or a salt thereof, a chelating agent, an enzyme other than the mutant protease of the present invention, an enzyme stabilizer, a fluorescence agent, a re-contamination inhibitor, a dispersant, a color transfer inhibitor, a finishing agent, a bleaching agent, an antioxidant, a solubilizer, a pH adjuster, a buffer, a preservative, a perfume, a salt, an alcohol, a sugar, and the like.

Examples of the water-soluble polymer include macromolecular compounds having a graft structure wherein the compound has (i) a polyether chain moiety containing epoxide-derived polymerization units having 2 to 5 carbon atoms and (ii) a polymer chain moiety containing polymerization units derived from an unsaturated carboxylic acid monomer selected from the group consisting of acrylic acid, methacrylic acid and maleic acid, where either (i) or (ii) is the main chain, and the other is a branched chain (JP-A-2010-275468, JP-A-1998-060496); and water-soluble polymers having alkylene terephthalate units and/or alkylene isophthalate units, and oxyalkylene units and/or polyoxyalkylene units (JP-A-2009-155606). The content of the water-soluble polymer in the detergent composition of the present invention is preferably from 0.2 to 10 mass%, and more preferably from 0.4 to 5 mass%.

Examples of the water-miscible organic solvent include alkylene glycols and glycerins of alkanols, polyalkylene glycols, (poly)alkylene glycol (mono or di)alkyl ethers, alkyl glyceryl ethers, and aromatic ethers of (poly)alkylene glycol. An alkylene glycol having 2 to 6 carbon atoms, such as ethylene glycol, propylene glycol, butylene glycol or hexylene glycol, glycerin, or polyethylene glycol monophenyl ether, ethylene glycol monobenzyl ether, diethylene glycol monobenzyl ether, or the like is preferable. The content of the water-miscible organic solvent in the detergent composition of the present invention is preferably from 1 to 40 mass%, and more preferably from 1 to 35 mass%.

Examples of the alkali agent include alkanolamines having 1 to 3 C2-C4 alkanols, such as monoethanolamine, diethanolamine, triethanolamine, polyoxyalkyleneamines, and dimethylaminopropylamine. Monoethanolamine and triethanolamine are preferable. The content of the alkali agent in the detergent composition of the present invention is preferably from 0 to 20 mass%, and more preferably from 0 to 10 mass%.

Examples of the organic acid or salt thereof include polyvalent carboxylic acids such as saturated fatty acids, succinic acid, maleic acid and fumaric acid, or salts thereof; and hydroxycarboxylic acids such as citric acid, malic acid, glycolic acid, p-hydroxybenzoic acid and benzoic acid, or salts thereof. Among them, citric acid or a salt thereof is preferable. The content of the organic acid or salt thereof in the detergent composition of the present invention is preferably from 0 to 5 mass%, and more preferably from 0 to 3 mass%.

The term "chelating agent" refers to a compound which can form a coordinate bond with a metal ion. The chelating agent added to the detergent composition has a blocking action on metal ions which have an adverse effect on cleaning, such as calcium ions and magnesium ions in laundry water or stains. Examples of the chelating agent to be contained in the detergent composition of the present invention include aminopolyacetic acids such as nitrilotriacetic acid, iminodiacetic acid, ethylenediamineacetic acid, diethylenetriaminepentaacetic acid, ethylene glycol tetraacetic acid, hydroxyethyliminodiacetic acid, triethylenetetraminehexaacetic acid and djenkolic acid, or salts thereof, and organic acids such as diglycollic acid, oxydisuccinic acid, carboxymethyloxysuccinic acid, citric acid, lactic acid, tartaric acid, oxalic acid, malic acid, oxydisuccinic acid, gluconic acid, carboxymethylsuccinic acid and carboxymethyltartaric acid, or salts thereof, aminotri(methylenephosphonic acid), 1-hydroxyethylidene-1,1-diphosphonic acid, ethylenediaminetetra(methylenephosphonic acid), diethylenetriaminepenta(methylenephosphonic acid), and alkali metals or lower amine salts thereof. The content of the chelating agent in the detergent composition of the present invention is preferably from 0.1 to 5 mass%, and more preferably from 0.1 to 4 mass%.

Examples of the re-contamination inhibitor and dispersant include polyacrylic acid, polymaleic acid, carboxymethylcellulose, polyethylene glycol having a weight average molecular weight of 5 000 or more, maleic anhydride-diisobutylene copolymers, maleic anhydride-methyl vinyl ether copolymers, maleic anhydride-vinyl acetate copolymers, naphthalenesulfonic acid salt formalin condensates, and polymers described in JP-A-1984-62614, claims 1 to 21 (page 1, paragraph 3, line 5 to page 3, paragraph 4, line 14). The contents of the re-contamination inhibitor and the dispersant in the detergent composition of the present invention are each preferably from 0.01 to 10 mass%.

Examples of the color transfer inhibitor include polyvinylpyrrolidone. The content thereof is preferably from 0.01 to 10 mass%.

As the bleaching agent, for example, hydrogen peroxide, a percarbonic acid salt, a perboric acid salt, or the like is preferably contained in the detergent composition at from 1 to 10 mass%. When the bleaching agent is used, tetraacetylethylenediamine (TAED) or a bleaching activator described in, for example, JP-A-1994-316700 may be contained in the detergent composition at from 0.01 to 10 mass%.

Examples of the fluorescence agent include biphenyl-type fluorescence agents (e.g., Tinopal CBS-X) and stilbene-type fluorescence agents (e.g., DM-type fluorescent dyes). The content of the fluorescence agent in the detergent composition of the present invention is preferably from 0.001 to 2 mass%.

Examples of the enzyme other than the mutant protease of the present invention include hydrolytic enzymes such as other proteases, cellulase, β-glucanase, hemicellulase, lipase, peroxidase, laccase, α-amylase, glucoamylase, cutinase, pectinase, reductase, oxidase, phenoloxidase, ligninase, pullulanases, pectate lyase, xyloglucanase, xylanase, pectin acetylesterase, polygalacturonase, rhamnogalacturonase, pectin lyase, mannanase, pectin methylesterase, cellobiohydrolase and transglutaminase, and mixtures of two or more thereof.

Examples of the enzyme stabilizer include boron compounds, calcium ion sources (calcium ion supply compounds), hydroxy compounds, and formic acid. Examples of the antioxidant include butylhydroxytoluene, distyrenated cresol, sodium sulfite and sodium hydrogen sulfite. Examples of the solubilizer include paratoluenesulfonic acid, cumenesulfonic acid, meta-xylenesulfonic acid, and benzoic acid salts (also effective as a preservative). The detergent composition of the present invention may further contain water-immiscible organic solvents such as paraffins such as octane, decane, dodecane and tridecane, olefins such as decene and dodecene, halogenated alkyls such as methylene chloride and 1,1,1-trichloroethane, terpenes such as D-limonene, pigments, perfumes, antibacterial preservatives, defoaming agents such as sili·BR>R-N, and the like.

Preferred examples of the detergent composition wherein the mutant protease of the present invention can be blended include a liquid detergent composition described in JP-A-2017-008303, and a liquid detergent composition described in JP-A-2013-129729. By blending the mutant protease of the present invention in such a detergent composition, the detergent composition of the present invention can be prepared.

Preferred examples of the detergent composition of the present invention include detergent compositions for clothing or cloth products (for example, sheets, curtains, carpets, wall-covering clothes and the like), and detergent compositions for kitchens (for example, eating utensils or cooking utensils), but the detergent composition of the present invention is not limited thereto. The detergent composition according to the present invention is preferably a detergent composition used for cleaning with a high-concentration detergent liquid, more preferably a detergent composition for application cleaning or immersion cleaning, and further more preferably a detergent composition for application cleaning.

The present invention encompasses the following substances, production methods, uses, methods and the like as exemplary embodiments. However, the present invention is not limited to these embodiments.

[1] A mutant protease consisting of the amino acid sequence of SEQ ID NO: 1 or an amino acid sequence having an identity of at least 90% with SEQ ID NO: 1, and containing at least one selected from the group consisting of the following amino acid residues (a) to (g):

(a) Arg or Lys at a position corresponding to position 191 of SEQ ID NO: 1;
(b) Arg or Lys at a position corresponding to position 17 of SEQ ID NO: 1;
(c) Ala or Phe at a position corresponding to position 141 of SEQ ID NO: 1;
(d) Arg or Lys at a position corresponding to position 243 of SEQ ID NO: 1;
(e) Arg or Lys at a position corresponding to position 300 of SEQ ID NO: 1;
(f) Arg or Lys at a position corresponding to position 302 of SEQ ID NO: 1; and
(g) Arg or Lys at a position corresponding to position 311 of SEQ ID NO: 1.

[2] The mutant protease according to [1], preferably containing the amino acid residue (a), and at least one selected from the group consisting of the amino acid residues (b) to (g).
[3] The mutant protease according to [1], preferably containing the amino acid residue (e), and the amino acid residue (f).
[4] The mutant protease according to [1], preferably containing at least one selected from the group consisting of the amino acid residues (a) to (g), and the following amino acid residue (h):
(h) Gln at a position corresponding to position 82 of SEQ ID NO: 1.
[5] The mutant protease according to [4], preferably containing the amino acid residue (a), and the amino acid residue (h).
[6] A polynucleotide encoding the mutant protease according to any one of [1] to [5].
[7] A vector containing the polynucleotide according to [6].
[8] A recombinant *Bacillus* bacterium containing the polynucleotide according to [6] or the vector according to [7].
[9] A method for producing a mutant protease using the recombinant *Bacillus* bacterium according to [8].
[10] The method according to [9], containing culturing the recombinant *Bacillus* bacterium.
[11] A detergent composition containing the mutant protease according to any one of [1] to [5].
[12] The detergent composition according to [11], which is preferably a detergent composition used for cleaning with a high-concentration detergent liquid, more preferably a detergent composition for application cleaning or immersion cleaning, and further more preferably a detergent composition for application cleaning.
[13] A method for producing a mutant protease, containing applying at least one selected from the group consisting of the following mutations (A) to (G) to a parent protease consisting of the amino acid sequence of SEQ ID NO: 1 or an amino acid sequence having an identity of at least 90% with SEQ ID NO: 1:

(A) substitution of Arg or Lys for an amino acid residue at position 191 of SEQ ID NO: 1 or a position corresponding thereto;
(B) substitution of Arg or Lys for an amino acid residue at position 17 of SEQ ID NO: 1 or a position corresponding thereto;
(C) substitution of Ala or Phe for an amino acid residue at position 141 of SEQ ID NO: 1 or a position corresponding thereto;
(D) substitution of Arg or Lys for an amino acid residue at position 243 of SEQ ID NO: 1 or a position corresponding thereto;
(E) substitution of Arg or Lys for an amino acid residue at position 300 of SEQ ID NO: 1 or a position corresponding thereto;
(F) substitution of Arg or Lys for an amino acid residue at position 302 of SEQ ID NO: 1 or a position corresponding thereto; and
(G) substitution of Arg or Lys for an amino acid residue at position 311 of SEQ ID NO: 1 or a position corresponding thereto.

[14] A method for improving the cleaning performance of a protease against protein stains in a high-concentration detergent liquid, containing applying at least one selected from the group consisting of the following mutations (A) to (G) to a parent protease consisting of the amino acid sequence of SEQ ID NO: 1 or an amino acid sequence having an identity of at least 90% with SEQ ID NO: 1:

(A) substitution of Arg or Lys for an amino acid residue at position 191 of SEQ ID NO: 1 or a position corresponding thereto;
(B) substitution of Arg or Lys for an amino acid residue at position 17 of SEQ ID NO: 1 or a position corresponding thereto;
(C) substitution of Ala or Phe for an amino acid residue at position 141 of SEQ ID NO: 1 or a position corresponding thereto;

(D) substitution of Arg or Lys for an amino acid residue at position 243 of SEQ ID NO: 1 or a position corresponding thereto;

(E) substitution of Arg or Lys for an amino acid residue at position 300 of SEQ ID NO: 1 or a position corresponding thereto;

(F) substitution of Arg or Lys for an amino acid residue at position 302 of SEQ ID NO: 1 or a position corresponding thereto; and

(G) substitution of Arg or Lys for an amino acid residue at position 311 of SEQ ID NO: 1 or a position corresponding thereto.

[15] The method according to [13] or [14], preferably containing applying the mutation (A), and at least one selected from the group consisting of the mutations (B) to (G) to the parent protease.

[16] The method according to [13] or [14], preferably containing applying the mutation (E) and the mutation (F) to the parent protease.

[17] The method according to [13] or [14], preferably containing applying at least one selected from the group consisting of the mutations (A) to (G), and the following mutation (H) to the parent protease:

(H) substitution of Gln for an amino acid residue at position 82 of SEQ ID NO: 1 or a position corresponding thereto.

Examples

Hereinafter, the present invention will be described in further detail with reference to Examples. However, the technical scope of the present invention is by no means limited to these Examples.

Example 1: Production of mutant protease

The wild-type KP43 protease expression plasmid pHA64TSA described in Patent Literature 5 at Reference Example 1 has a tetracycline resistance gene, and the full length of the coding sequence of the wild-type KP43 protease connected to a promoter region derived from the alkaline cellulase K-64 gene of the *Bacillus* sp. KSM-64 strain. With pHA64TSA as a template, a site-directed mutation was repeatedly introduced into the KP43 protease gene by PCR-based site-directed introduction with a pair of complementary primers (see Nucleic Acids Research, 2004, 32(14): e115) to produce a plasmid expressing a protease of SEQ ID NO: 1. By a similar method using the obtained plasmid expressing the protease of SEQ ID NO: 1 as a template, plasmids expressing a mutant protease having the following amino acid substitution: T311R, N302R, Y300K, N243K, S191R, Y17R, D141A, D141F, Y17R/S191R, S191R/N243K, Y300K/N302R, E82Q/S191R or S191F relative to the protease of SEQ ID NO: 1 as a parent protease were produced. Hereinafter, the mutant proteases expressed from these plasmids are referred to as T311R, N302R, Y300K, N243K, S191R, Y17R, D141A, D141F, Y17R/S191R, S191R/N243K, Y300K/N302R, E82Q/S191R and S191F, respectively.

With the produced plasmids expressing the parent enzyme or the mutant protease, host bacteria of the *Bacillus* sp. KSM9865 strain (FERM P-18566) were transformed. The transformation was carried out by an electroporation method, and cells after the transformation were applied to an alkaline agar culture medium containing fat-free milk [1% fat-free milk (Difco Laboratories), 1% Bacto Tryptone (Difco Laboratories), 0.5% yeast extract (Difco Laboratories), 1% sodium chloride, 1.5% agar, 0.05% sodium carbonate and 15 ppm tetracycline; % means (w/v)%], and cultured at 30°C for several days. Among the colonies grown on the agar culture medium, those having spots of dissolved fat-free milk were selected as a transformant having a protease gene introduced. The obtained transformant was seeded in 5 mL of a seed culture medium [6.0% polypeptone S, 0.05% yeast extract, 1.0% maltose, 0.02% magnesium sulfate heptahydrate, 0.1% potassium dihydrogen phosphate, 0.25% sodium carbonate, and 30 ppm tetracycline; % means (w/v)%], and cultured under shaking conditions at 30°C for 16 hours. Then, the seed culture solution was seeded at 1% (v/v) in 20 mL of a primary culture medium [8% polypeptone S, 0.3% yeast extract, 10% maltose, 0.04% magnesium sulfate heptahydrate, 0.2% potassium dihydrogen phosphate, 1.5% anhydrous sodium carbonate and 30 ppm tetracycline; % means (w/v)%], and cultured under shaking conditions at 30°C for 3 days. The obtained culture solution was centrifuged to obtain a culture supernatant containing a protease. The concentration of protein contained in the culture supernatant was measured by using Protein Assay Rapid Kit Wako II (FUJIFILM Wako Pure Chemical Corporation).

Example 2: Evaluation of application cleaning power of mutant protease

A commercially available concentrated liquid detergent (Attack ZERO Regular; Kao Corporation) was heated in a microwave oven to deactivate the enzyme in the product. To the liquid detergent, each of the culture supernatants obtained in Example 1, which contained the respective mutant proteases, was added to a protein concentration of 0.1 g/L. The mixture was thoroughly stirred to prepare a liquid detergent composition containing a mutant protease (a test composition). By a similar procedure, a liquid detergent composition containing a parent enzyme (a control composition) was prepared as a control. Using the obtained liquid detergent composition, application cleaning was carried out on an artificially contaminated cloth DINGY_TN/CP/(CONSUMERTEC) which is a collar/sleeve stain model. Specifically, 20 μL of the test composition or the control composition was dropped onto the DINGY_TN/CP/ cut into a square with sides 1.5 cm, and was left to stand for 5 minutes, and the contaminated cloth was then transferred into 600 mL of tap water maintained at 20°C in the cleaning tank of Tergotometer (Ueshima Seisakusho Co., Ltd.), and cleaned at 60 rpm for 10 minutes. The contaminated cloth after the application cleaning was taken out from the cleaning tank, rinsed with tap water, and dried. The lightness values (L values) of the contaminated cloth before and after the application cleaning, and a separately provided cloth identical to the contaminated cloth except for being uncontaminated were measured using a spectral colorimeter cm-700d (Konica Minolta, Inc.), and the cleaning efficiency of the contaminated cloth by the following equation.

$$\texttt{Cleaning efficiency (\%) = (L_2-L_1)/(L_0-L_1) \times 100}$$

($L_0$: L value of cloth identical to contaminated cloth except for being uncontaminated, $L_1$: L value of contaminated cloth before cleaning, $L_2$: L value of contaminated cloth after cleaning).

The relative cleaning efficiency of each test composition compared to the control composition (cleaning efficiency with test composition/cleaning efficiency with control composition) was calculated, and the relative cleaning power (relative application cleaning power) of each mutant protease compared to the parent protease in application cleaning was evaluated.

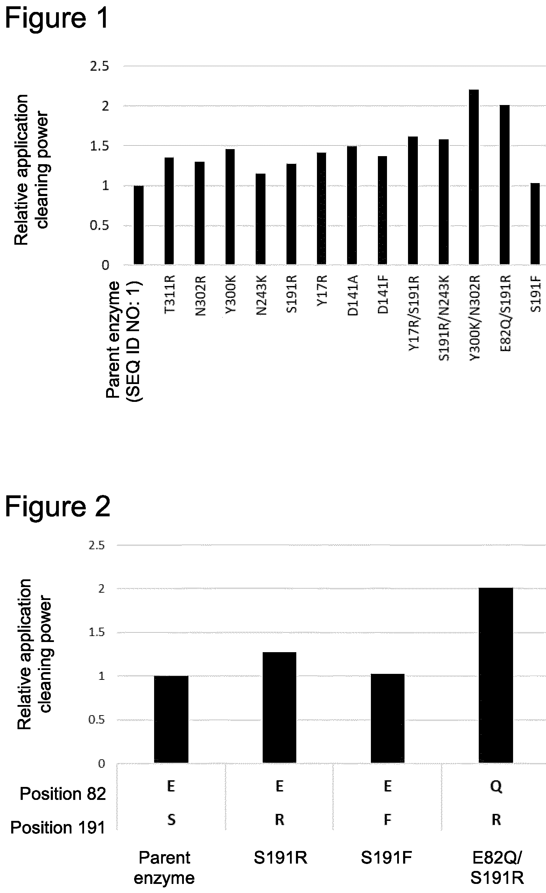

The relative application cleaning power of each mutant protease (relative cleaning efficiency of each of test compositions containing respective mutant proteases) is shown in Table 2 and Figure 1. All mutant proteases, except for S191F, had a relative application cleaning power of more than 1, and exhibited obviously higher application cleaning power over the parent enzyme.

[Table 2]

| Enzyme | Relative application cleaning power |
|---|---|
| Parent enzyme (SEQ ID NO: 1) | 1 |
| T311R | 1.35 |
| N302R | 1.30 |
| Y300K | 1.46 |
| N243K | 1.15 |
| S191R | 1.27 |
| Y17R | 1.41 |
| D141A | 1.49 |
| D141F | 1.36 |
| Y17R/S191R | 1.61 |
| S191R/N243K | 1.58 |
| Y300K/N302R | 2.21 |
| E82Q/S191R | 2.01 |
| S191F | 1.03 |

The effects of mutations at position 191 relative to the parent protease of SEQ ID NO: 1 are shown in Table 3 and Figure 2. A protease obtained by substituting arginine (R) for serine (S) at position 191 of SEQ ID NO: 1 (S191R) had improved application cleaning power as compared to the parent protease. On the other hand, the substitution of phenylalanine (F) at position 191 (S191F) did not have an effect on application cleaning power. A mutant having glutamine at position 82 and arginine at position 191 (E82Q/S191R) exhibited further high application cleaning power.

[Table 3]

| Enzyme | Position 191 | Position 82 | Relative application cleaning power |
|---|---|---|---|
| Parent enzyme (SEQ ID NO: 1) | S | E | 1 |
| S191R | R | E | 1.27 |
| S191F | F | E | 1.03 |
| E82Q/S191R | R | Q | 2.01 |

Example 3: Evaluation of stability of mutant protease

A commercially available concentrated liquid detergent (Attack ZERO Regular; Kao Corporation) was heated in a microwave oven to deactivate the enzyme in the product. To the liquid detergent, the culture supernatant containing the mutant protease E82Q/S191R obtained in Example 1, was added to a protein concentration of 0.2 g/L. The mixture was thoroughly stirred to prepare a liquid detergent composition containing a mutant protease (a test sample). By a similar procedure, a liquid detergent composition containing a parent enzyme (a control sample) was prepared as a control. Immediately after blending of the enzyme (0 h) and after storage at 40°C for 2 weeks (2 w), the protease activity of the test sample was measured by the following method. One tablet of Protazyme AK Tablets (Megazyme) was suspended in 1 mL of 0.1 M phosphate buffer to obtain a substrate solution. At each of 0 h and 2 w, the test sample was diluted 60-fold with 0.1 M phosphate buffer. To the substrate solution was added 1 mL of the diluted sample, followed by incubation at 40°C for 30 minutes. 10 mL of a 2% trisodium phosphate aqueous solution was added thereto to terminate the reaction, the mixture was stirred, and then the reaction liquid was put in 1.5 mL tubes with each tube containing 1 mL of the reaction liquid. The reaction liquid was centrifuged at 12 000 rpm for 5 minutes, and the supernatant was taken, followed by measurement of an absorbance at 590 nm. Using the absorbance similarly measured using a thermally deactivated liquid detergent free of the enzyme instead of the test sample as a blank, a difference between the absorbance of the test sample and the blank was taken as an activity value. By a similar procedure, the activity values of the control sample at 0 h and 2 w were determined. The activity value of the sample at 2 w was divided by the activity value at 0 h to determine a residual activity rate after two weeks, and the value was raised to the power of 1/14 to determine an activity retention rate per day under treatment at 40°C. A logarithm of 0.5 to the base of the activity retention rate per day was determined, and taken as a half-life (days) of the protease activity. The half-life of the mutant protease was divided by the half-life of the parent protease to determine relative stability. The relative stability of the mutant E82Q/S191R was 1.89, and the stability of the protease was significantly improved by introduction of the mutation of E82Q and S191R. Patent Literature 3 indicates that a protease having 82E or 82Q has high stability in liquid detergent. On the other hand, the mutant E82Q/S191R of the present Example has improved stability as compared to the parent protease having 82E, which suggests that the mutation of S191R further improves the stability of the protease.

The embodiments of the present invention have been described above, however, it should be understood that the present invention is by no means restricted to the described specific embodiments. Other various changes and modifications within the scope of the present invention are obvious to those skilled in the art.

Documents and patent applications cited in the present specification are incorporated by reference as if they were completely described herein in their entirety.

**Claims**

1. A mutant protease consisting of an amino acid sequence of SEQ ID NO: 1 or an amino acid sequence having an identity of at least 90% with SEQ ID NO: 1 and comprising at least one selected from the group consisting of the following amino acid residues (a) to (g):

   (a) Arg or Lys at a position corresponding to position 191 of SEQ ID NO: 1;
   (b) Arg or Lys at a position corresponding to position 17 of SEQ ID NO: 1;
   (c) Ala or Phe at a position corresponding to position 141 of SEQ ID NO: 1;
   (d) Arg or Lys at a position corresponding to position 243 of SEQ ID NO: 1;
   (e) Arg or Lys at a position corresponding to position 300 of SEQ ID NO: 1;
   (f) Arg or Lys at a position corresponding to position 302 of SEQ ID NO: 1; and
   (g) Arg or Lys at a position corresponding to position 311 of SEQ ID NO: 1.

2. The mutant protease according to Claim 1, comprising the amino acid residue (a) and at least one selected from the group consisting of the amino acid residues (b) to (g).

3. The mutant protease according to Claim 1, comprising the amino acid residue (e) and the amino acid residue (f).

4. The mutant protease according to Claim 1, comprising at least one selected from the group consisting of the amino acid residues (a) to (g), and the following amino acid residue (h):
(h) Gln at a position corresponding to position 82 of SEQ ID NO: 1.

5. The mutant protease according to Claim 4, comprising the amino acid residue (a), and the amino acid residue (h).

6. A polynucleotide encoding the mutant protease according to any one of Claims 1 to 5.

7. A vector comprising the polynucleotide according to Claim 6.

8. A recombinant *Bacillus* bacterium comprising the polynucleotide according to Claim 6 or the vector according to Claim 7.

9. A method for producing a mutant protease, which uses the recombinant *Bacillus* bacterium according to Claim 8.

10. A detergent composition comprising the mutant protease according to any one of Claims 1 to 5.

11. The detergent composition according to Claim 10, for application cleaning.

12. A method for producing a mutant protease, comprising applying, to a parent protease consisting of an amino acid sequence of SEQ ID NO: 1 or an amino acid sequence having an identity of at least 90% with SEQ ID NO: 1, at least one selected from the group consisting of the following mutations (A) to (G):

    (A) substitution of Arg or Lys for an amino acid residue at position 191 of SEQ ID NO: 1 or a position corresponding thereto;
    (B) substitution of Arg or Lys for an amino acid residue at position 17 of SEQ ID NO: 1 or a position corresponding thereto;
    (C) substitution of Ala or Phe for an amino acid residue at position 141 of SEQ ID NO: 1 or a position corresponding thereto;
    (D) substitution of Arg or Lys for an amino acid residue at position 243 of SEQ ID NO: 1 or a position corresponding thereto;
    (E) substitution of Arg or Lys for an amino acid residue at position 300 of SEQ ID NO: 1 or a position corresponding thereto;
    (F) substitution of Arg or Lys for an amino acid residue at position 302 of SEQ ID NO: 1 or a position corresponding thereto; and
    (G) substitution of Arg or Lys for an amino acid residue at position 311 of SEQ ID NO: 1 or a position corresponding thereto.

13. A method for improving the cleaning performance of a protease against protein stains in a high-concentration detergent liquid, comprising applying, to a parent protease consisting of an amino acid sequence of SEQ ID NO: 1 or an amino acid sequence having an identity of at least 90% with SEQ ID NO: 1, at least one selected from the group consisting of the following mutations (A) to (G):

    (A) substitution of Arg or Lys for an amino acid residue at position 191 of SEQ ID NO: 1 or a position corresponding thereto;
    (B) substitution of Arg or Lys for an amino acid residue at position 17 of SEQ ID NO: 1 or a position corresponding thereto;
    (C) substitution of Ala or Phe for an amino acid residue at position 141 of SEQ ID NO: 1 or a position corresponding thereto;
    (D) substitution of Arg or Lys for an amino acid residue at position 243 of SEQ ID NO: 1 or a position corresponding thereto;
    (E) substitution of Arg or Lys for an amino acid residue at position 300 of SEQ ID NO: 1 or a position corresponding thereto;
    (F) substitution of Arg or Lys for an amino acid residue at position 302 of SEQ ID NO: 1 or a position corresponding thereto; and

(G) substitution of Arg or Lys for an amino acid residue at position 311 of SEQ ID NO: 1 or a position corresponding thereto.

14. The method according to Claim 12 or 13, comprising applying the mutation (A), and at least one selected from the group consisting of the mutations (B) to (G) to the parent protease.

15. The method according to Claim 12 or 13, comprising applying the mutation (E) and the mutation (F) to the parent protease.

16. The method according to Claim 12 or 13, comprising applying at least one selected from the group consisting of the mutations (A) to (G), and the following mutation (H) to the parent protease:

(H) substitution of Gln for an amino acid residue at position 82 of SEQ ID NO: 1 or a position corresponding thereto.

# Figure 1

# Figure 2

## INTERNATIONAL SEARCH REPORT

| International application No. |
| --- |
| **PCT/JP2023/025933** |

### A. CLASSIFICATION OF SUBJECT MATTER

*C12N 15/57*(2006.01)i; *C11D 7/42*(2006.01)i; *C12N 1/21*(2006.01)i; *C12N 9/50*(2006.01)i; *C12N 15/63*(2006.01)i; *C12P 21/02*(2006.01)i

FI: C12N15/57; C12N15/63 Z; C12N1/21; C12P21/02 C; C11D7/42; C12N9/50 ZNA

According to International Patent Classification (IPC) or to both national classification and IPC

### B. FIELDS SEARCHED

Minimum documentation searched (classification system followed by classification symbols)

C12N15/57; C11D7/42; C12N1/21; C12N9/50; C12N15/63; C12P21/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); UniProt/GeneSeq

### C. DOCUMENTS CONSIDERED TO BE RELEVANT

| Category* | Citation of document, with indication, where appropriate, of the relevant passages | Relevant to claim No. |
| --- | --- | --- |
| Y | WO 2020/184410 A1 (KAO CORP) 17 September 2020 (2020-09-17) claims, paragraph [0006], example 5 | 1-16 |
| Y | WO 2017/213168 A1 (KAO CORP) 14 December 2017 (2017-12-14) claims, paragraph [0005], example 2 | 1-16 |

☐ Further documents are listed in the continuation of Box C.   ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **12 September 2023** | **19 September 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2023/025933**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    The "form of an Annex C/ST.25 text file" is replaced with the "form of ST.26."

Form PCT/ISA/210 (continuation of first sheet) (January 2015)

## INTERNATIONAL SEARCH REPORT
### Information on patent family members

International application No.

**PCT/JP2023/025933**

| Patent document cited in search report | | | Publication date (day/month/year) | Patent family member(s) | | | Publication date (day/month/year) |
|---|---|---|---|---|---|---|---|
| WO | 2020/184410 | A1 | 17 September 2020 | US | 2022/0154109 | A1 | |
| | | | | claims, paragraphs [0013]-[0019], example 5 | | | |
| | | | | EP | 3940050 | A1 | |
| | | | | CN | 113557292 | A | |
| WO | 2017/213168 | A1 | 14 December 2017 | US | 2019/0161708 | A1 | |
| | | | | claims, paragraphs [0005]-[0012], example 2 | | | |
| | | | | EP | 3470517 | A1 | |
| | | | | CN | 109312323 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9918218 A
- JP 2008212084 A
- JP 2010273672 A
- JP 2013233141 A
- JP 2020145938 A
- JP 3492935 B
- JP 2010275468 A
- JP 2010265445 A
- JP 2011063784 A
- JP 2010189551 A
- JP 2010229385 A
- JP 2010229387 A
- JP 2010254881 A
- JP 2010265333 A
- WO 9824865 A
- JP 8157867 A
- JP 2008007706 A
- JP 2009007451 A
- JP 2009155594 A
- JP 2009155606 A
- JP 2009144002 A
- JP 2009173858 A
- JP 2010189612 A
- JP 10060496 A
- JP 59062614 A
- JP 6316700 A
- JP 2017008303 A
- JP 2013129729 A

### Non-patent literature cited in the description

- *Science*, 1975, vol. 227, 1435-1441
- **THOMPSON, J. D. et al.** *Nucleic Acids Res.*, 1994, vol. 22, 4673-4680
- **SAEKI et al.** *Journal of bioscience and Bioengineering*, 2007, vol. 103, 501-508
- **MATSUMURA et al.** Shin Idenshi Kogaku Handbook, the revised fourth edition. Yodosha Co., Ltd., 82-88
- **HORTON et al.** *Gene*, 1989, vol. 77 (1), 61-68
- *Nucleic Acids Research*, 1989, vol. 17, 7059-7071
- **ISHIKAWA** ; **SHIBAHARA**. *Jpn J Genet*, 1985, vol. 60, 235-243
- **MORIYAMA et al.** *Nucleic Acids Res*, 1988, vol. 16, 8732
- **GRYCZAN et al.** *J Bacteriol*, 1978, vol. 134, 318-329
- **BRON et al.** *Plasmid*, 1987, vol. 18, 8-15
- **YAMANE et al.** Formation of Fused Extracellular Thermostable α-amylase Using Bacillus subtilis Secretion Vector. *Journal of the Japanese Society of Starch Science*, 1987, vol. 34, 163-170
- *J Bacteriol*, 1967, vol. 93, 1925-1937
- *FEMS Microbiol Lett*, 1990, vol. 55, 135-138
- *Mol Gen Genet*, 1979, vol. 168, 111-115
- *J Bacteriol*, 1983, vol. 156, 1130-1134
- **SAMBROOK et al.** Molecular Cloning, A laboratory manual. Cold Spring Harbor Laboratory Press, 2001
- **KOSOKIN KEI** ; **HIROHUMI YOSHIKAWA**. *Zoku Seikagaku Zikken Koza 1. Idenshi Kenkyu Ho II*, 1986, 150-169
- *Nucleic Acids Research*, 2004, vol. 32 (14), e115